(19) Europäisches Patentamt
European Patent Office
Office européen des brevets

(11) Veröffentlichungsnummer: **0 188 749 B1**

(12) # EUROPÄISCHE PATENTSCHRIFT

(45) Veröffentlichungstag der Patentschrift: **06.03.91**

(51) Int. Cl.5: **C07C 69/60**, A61K 31/225, C07C 67/14

(21) Anmeldenummer: **85116011.9**

(22) Anmeldetag: **16.12.85**

(54) **Fumarsäurederivate, Verfahren zu ihrer Herstellung und sie enthaltende pharmazeutische Zubereitungen.**

(30) Priorität: **15.01.85 CH 161/85**

(43) Veröffentlichungstag der Anmeldung:
**30.07.86 Patentblatt 86/31**

(45) Bekanntmachung des Hinweises auf die Patenterteilung:
**06.03.91 Patentblatt 91/10**

(84) Benannte Vertragsstaaten:
**AT BE CH DE FR GB IT LI LU NL SE**

(56) Entgegenhaltungen:
**EP-A- 0 071 412**
**EP-A- 0 129 778**
**FR-A- 1 426 959**
**GB-A- 928 263**

(73) Patentinhaber: **Speiser, Peter Paul, Prof. Dr.**
**Freudenbergstrasse 101/D2**
**CH-8044 Zürich(CH)**

Patentinhaber: **Joshi, Rajendra K., Dr.**
**Altstetterstrasse 224**
**CH-8048 Zürich(CH)**

(72) Erfinder: **Speiser, Peter Paul, Prof. Dr.**
**Freudenbergstrasse 101/D2**
**CH-8044 Zürich(CH)**
Erfinder: **Joshi, Rajendra K., Dr.**
**Altstetterstrasse 224**
**CH-8048 Zürich(CH)**

(74) Vertreter: **Dipl.-Ing. Schwabe, Dr. Dr. Sandmair, Dr. Marx et al**
**Stuntzstrasse 16**
**W-8000 München 80(DE)**

**EP 0 188 749 B1**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Rank Xerox (UK) Business Services

**Beschreibung**

Die Erfindung betrifft neue Fumarsäurederivate, Verfahren zu ihrer Herstellung und sie enthaltende pharmazeutische Zubereitungen.

Pharmazeutische Zubereitungen, die nach Verabreichung bei ihrem biologischen Abbau in den Zitronensäurezyklus einmünden oder diesem angehören, gewinnen meist in hoher Dosierung, immer mehr an therapeutischem Wert, weil man mit ihrer Hilfe kryptogenetisch bedingte Krankheiten zu lindern oder heilen vermag. So hemmt Fumarsäure das Wachstum des Ehrlich-Ascitestumors bei Mäusen, vermindert die toxischen Effekte von Mitomycin C und Aflatoxin (Kuroda, K.M.Akao, Biochem.Pharmacol. 29 , 2839-2844, 1980/Gann. 72 , 777-782, 1981/Ca-ncer Res. 36 , 1900-1903, 1976) und besitzt eine antipsoriatische Wirkung (Schweckendiek W., Med. Mschr. 13 , 103-104, 1959/Dubiel, W., R. Happle, Z.Haut- u. Geschl.krkht. 47 , 545-550, 1972/Schäfer, G., Selecta 17 , 1868-1872, 1982) sowie antimikrobielle Wirkung (C.H. Huhtsnen, J. Food Sci., 48 , 1574 (1983), M.N. Islam, U.S. Pat. 4 346 118 v. 24.8.1982/C.A., 97 , 161317b (1982)).

Hohe Verabreichungsdosen von Fumarsäure, ihren Salzen oder ihrer bisher bekannten Derivate wie Dihydroxyfumarsäure, Fumaramid und Fumaronitril besitzen bei parenteraler, dermaler, insbesondere aber peroraler Verabreichung eine derart unzumutbare Nebenwirkungsrate und hohe Toxizität (Raab, W., Z. Hautkr. 59 , 671-679, 1984/Holland P., R.G. White, Brit.J.Dermatol. 85 , 259-263, 1971/Hagedorn M., K.W. Kalkoff, G. Kiefer, D. Baron, J. Hug, J. Petres, Arch.Derm.Res. 254 , 67-73, 197), daß bisher meist von einer solchen Therapie abgesehen werden mußte.

Die erfindungsgemäßen neuen Fumarsäurederivate und sie enthaltende pharmazeutische Zubereitungen enthalten ambiphile Voll- oder Halbester der Fumarsäure. Dabei ist einerseits die eine Carboxylgruppe der Fumarsäure mit einem Lipid, einem langkettigen aliphatischen Fettalkohol mit einer Kettenlänge zwischen 6 und 24 Kohlenstoffatomen verestert, die andere Carboxylgrüppe der Fumarsäure ist mit einem niedrigen aliphatischen Alkohol mit 1 bis 8 Kohlenstoffatomen, der verschieden vom ersten Alkohol ist, verestert.

Die bisher bekannten Salze und Derivate der Fumarsäure wurden aufgrund ihres verhältnismäßig stark polaren, hydrophilen Charakters und während ihrer kurzen Verweildauer an lipophilen Organgrenzschichten nicht oder nur ungenügend resorbiert. Aus diesem Grunde müssen hohe Dosen eingesetzt werden, die zu vielen Nebenwirkungen (Aufstoßen, Schwindel, Ubelkeit, Erbrechen, Magen- und Darmkrämpfe, Flush Syndrom) Anlaß gaben.

Die erfindungsgemäßen Fumarsäurederivate stellen ambiphile (amphiphile) Prodrugs dar, die grenzflächenaktive Eigenschaften besitzen, gut resorbierbar und verteilbar sind, im Organismus kontrolliert In das aktive Fumaratanion umgewandelt werden und überraschenderweise die bisher bekannten Nebenwirkungen nicht aufweisen. Die erfindungsgemäßen Verbindungen sind wesentlich verträglicher, weisen die oben erwähnten Nebenwirkungen nicht mehr auf und werden aufgrund ihrer lipophilen bis ambiphilen Charakters besser resorbiert. Durch die erfindungsgemäßen neuen Prodrug-Systeme werden dem Gesamtmolekül lipophile und hydrophile Eigenschaften zugleich erteilt, wodurch eine bessere Membran- oder Grenzschichtpassage ermöglicht wird. Durch Variation der Substituenten kann die Ambiphilie (HLB, Verteilungskoeffizient) des Gesamtmoleküls besser auf eine optimale Membranpassage des jeweils angesteuerten Zielorgans eingestellt werden.

Die erfindungsgemäße Verwendung apolarer und schwach polarer Substituenten gewährleistet nicht nur eine optimale Resorption und Verteilung, sondern auch eine kontrollierte und steuerbare Freisetzung des Fumaratanions durch Esterhydrolyse mit Hilfe körpereigener Enzymsysteme, ohne daß bei ihrem Abbau Bruchstückmoleküle oder Metaboliten mit physiologisch unerwünschten Eigenschaften entstehen.

Bei den erfindungsgemäßen Fumarsäurederivaten ist die Fumarsäure ganz oder partiell an'Lipide (Fettalkohol), mit einer Kettenlänge von 6 bis 24 C-Atomen gebunden. Durch Wahl des Lipidanteils kann die Lipophilie bis Ambiphilie des Gesamtmoleküls gesteuert werden. Beispiele geeigneter Lipide sind Valerylalkohol, Caprylalkohol, Laurylalkohol, Palmitylalkohol, Stearylalkohol, Cetylalkohol, Oleylalkohol, Linoleylalkohol, Ricinoylalkohol.

Die vorliegende Erfindung betrifft:

Fumarsäurederivate der allgemeinen Formel (I)

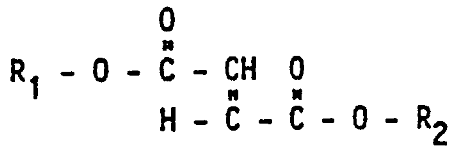

(I)

in der

$R_1$ ungleich $R_2$ ist (unsymmetrisch) und

$R_1$ eine $C_1$ - $C_8$-Alkylgruppe oder ein metallisches Kation wie zu Beispiel Ca, Zn und (außer Ca-Salz von Verbindungen, wo $R_2$ $C_{14}$, $C_{16}$ oder $C_{18}$ ist)

$R_2$ eine gesättigte oder ungesättigte aliphatische $C_6$ - $C_{24}$-Kohlenwasserstoffgruppe bedeuten.

Die vorliegende Erfindung betrifft auch:

Verfahren zur Herstellung von Fumarsäurederivaten gemäß der obigen allgemeinen Formel (I), bei dem man eine Verbindung der allgemeinen Formel (II)

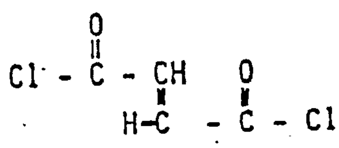

(II)

a) mit 2 Mol Alkylalkohol ($R_2OH$) zum Diester kondensiert und anschließend kontrolliert zum Monoester hydrolisiert, oder

b) mit 1 Mol eines entsprechenden Alkylalkohols ($R_2OH$) kondensiert und das erhaltene Monosäurechlorid zur Säure hydrolisiert oder

c) Fumarsäure direkt mit 2 Mol Alkylalkohol ($R_2OH$) gemäß Anspruch 1 zu einem Diester kondensiert und anschließend kontrolliert zum Monoester hydrolysiert, oder

d) der in a), b) und c) hydrolysierte Monoester wird zu der Verbindung der obigen Formel (1) aufgeführten Salzen umgesetzt.

Die vorliegenden Erfindung betrifft ferner:

Verfahren zur Herstellung von Fumarsäurederivaten gemäß obiger Formel (I) bei dem man eine Verbindung der allgemeinen Formel (III)

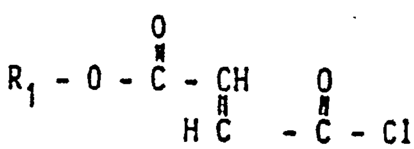

(III),

in der $R_1$ die vorstehend angegebene Bedeutung hat, mit einem Mol eines entsprechenden Alkylalkohols ($R_2OH$) unter Bildung eines unsymmetrischen Diesters der Gruppierung $R_2$ gemäß obiger Formel (I) kondensiert.

Gegenstand der Erfindung sind auch:

Pharmazeutische Zubereitungen, enthaltend Fumarsäurederivate der allgemeinen Formel (I) für die orale Verabreichung in Form von Kapseln, Granulaten und Tabletten, für die parenterale Verabreichung in Form wässriger Mikro-Dispersionen, O/W-Emulsionen oder ölige Lösungen, für die rektale Verabreichung als Suppositorien oder Mikroklistiere und pharmazeutische Zubereitungen, enthaltend Fumarsäurederivate der allgemeinen Formel (I) wie in Anspruch 11 definiert, für die kutane und transdermale Verabreichung gegen Psoriasis in Form von Salben, Pflastern Lotionen und Duschmitteln, sowie für die Behandlung von Haaren, Finger- und Zehennägeln.

Beispiel 1

Herstellung von Cetylethylfumarat (Vollester)

60,61 g (0,25 Mol) 1-Hexadecanol (MG 242,45) werden in 300 ml ethanolfreiem Chloroform und 25 ml absolutem Pyridin (0,31 Mol) gelöst und durch tropfenweise Zugabe eine Lösung von 44,7 g Fumarsäuremonoethylesterchlorid (0,275 Mol , MG 162, 45) in 100 ml gereinigtem Chloroform auf konstant 35 °C gehalten. Nach beendeter Umsetzung wird das violette Reaktionsgemisch mit 800 ml Petrolether verdünnt und durch Waschen mit je 100 ml Wasser, 2 mal 1 N Salzsäure, Wasser, 5% $Na_2CO_3$ und Wasser gereinigt, getrocknet und das Lösungsmittel entfernt (RV). Der feste Rückstand wurde aus Petrolether oder 94 % Ethanol mehrmals umkristallisiert, wobei insgesamt 70 g Produkt entspr. 79 % der theoretischen Ausbeute erhalten wurden ( $C_{22}H_{40}O_4$, MG 368,56) Smp. 32 °C.

Analyse
Für die Bruttoformel: $C_{22}H_{40}O_4$
Berechnet: C 71 ,7, H 10, 9, O 17, 4
gefunden: C 71 ,7, H 10 ,9, O 17 ,5

Beispiel 2

Herstellung von Ethyl-octadecyl-fumarat (Vollester)

Es wurden 135 g (0,5 Mol) 1-Octadecanol und 65 ml (0,55 Mol) Chinolin unter Rühren bei 40 °C in 500 ml ethanolfreiem Chloroform gelöst. Der klaren Lösung wurden während 1 Stunde 89,4 g (0,55 Mol) Fumarsäuremonoethylesterchlorid zugetropft und 2 Stunden am Rückfluß gekocht. Die Reaktionsmischung wurde mit 500 ml Petrolether verdünnt und 2 mal mit 0,5 N Salzsäure, Wasser 5 % Kaliumcarbonatlösung und Wasser ausgeschüttelt, über Natriumsulfat getrocknet und unter vermindertem Druck eingedampft. Der feste Rückstand wurde aus 95 % Ethanol umkristallisiert. Ausbeute: 139 g Produkt mit einem Schmelzpunkt von 42 °C.

Analyse
Für die Bruttoformel: $C_{24}H_{44}O_4$
Berechnet: C 72,7, H 11,2, O 16 ,1
gefunden: C 73,0, H 11,4, O 16,2

Beispiel 3

Herstellung von Octyl-hydrogenfumarat (Halbester)

Eine Lösung von 72,78 g abs. Pyridin (0,92 Mol, MG 79,1), 119,81 g Octanol (0,92 Mol, MG 130,23) in 120 ml ethanolfreiem Chloroform wird mit 70,6 g Fumarsäuredichlorid (0,46 Mol, MG 152,97) unterkühlt, bei Raumtemperatur zum Dioctylester umgesetzt, mit Petrolether auf 750 ml verdünnt und durch Waschen mit 0,1 N HCl, Wasser, 5 % $Na_2CO_3$-Lösung und Wasser gereinigt, getrocknet, filtriert und das Lösungsmittel am Rotationsverdampfer entfernt. Das Produkt wurde bei 0,46 mbar (0,35 Torr) und 175 °C durch Destillation gereinigt. Die Hydrolyse zum Monooctylester erfolgt am Rückfluß in einer Kaliumhydroxidlösung im 60%igen Ethanol während 2 Stunden. Nach Zugabe von Wasser wird mit Petrolether gereinigt, die wäßrige Phase mit verd. Salzsäurelösung auf pH 4 eingestellt und 3 mal mit Chloroform extrahiert. Die vereinigten Chloroformextrakte werden getrocknet, filtriert, eingedampft und aus Petrolether auskristallisiert.

(Smp 670°). Eine 0,1%ige Lösung in Miglyol[R] 810 gelöst, ergibt mit der Ringmethode gegen Wasser eine Grenzflächenspannung von 6,1 $mN^{-1}$.

Analyse

Für die Bruttoformel: $C_{12}H_{20}O_4$

berechnet: C 63,1, H 8,8, O 28,0

gefunden: C 62,9, H 8,8, O 28,1

Beispiel 4

Herstellung von Dodecyl-hydrogenfumarat (Halbester)

122,3 g (0,8 Mol) Fumarsäuredichlorid in 100 ml Benzol p.a. wurden auf 65° C erwärmt. Zu der erwärmten Lösung wurde langsam während 3 Stunden 149 g (0,8 Mol) Dodecanol, gelöst in 300 ml Benzol p.a., zugetropft und bei 80° C weitere 10 Stunden gerührt. Unter vermindertem Druck wurden das Lösungsmittel und das verbliebene Fumarsäuredichlorid entfernt. Der Rückstand wurde zur Säurechloridhydrolyse mit einer Aceton-Wasser-Mischung versetzt. Der weiße Niederschlag wurde abfiltriert, mit Wasser gründlich gewaschen, getrocknet und aus Petrolether oder 80 % Ethanol umkristallisiert. Ausbeute: 120 g Produkt mit einem Schmelzpunkt von 78,4° C.

Beispiel 5

Herstellung von Kapseln enthaltend 150 mg Cetyl-ethylfumarat und 80 mg Fumarsäure-monoethylester Calciumsalz

15,0 kg Cetyl-ethyl-Fumarat und 8,0 kg Fumarsäure-monoethyl-ester Ca-Salz werden in einem Gemisch von 21 kg Lactose, 0,5 kg Magnesiumstearat, 1,0 kg kolloidaler Kieselsäure (Aerosil [R]) mit einer 2%igen wässrigen Lösung von Polyvinylprrolidon (PVP, Kollidon K30 [R]) feucht granuliert (Sieb 800) und getrocknet. Dann wird 0,5 kg FST-Komplex als äußere Phase zugemischt und je 460 mg des gut fließenden Granulates in Hartgelatinekapseln der Größe Nr. 00 gefüllt Die Granulateigenschaften wie Korngröße (mittels Siebanalyse bestimmt), Fließeigenschaften, Schüttdichte, Stampfvolumen und relative Verdichtung sowie die Dosierungsgenauigkeit und Gleichförmigkeit der Füllmasse in den Kapseln entsprechen dabei den Anforderungen der Arzneibücher.

Beispiel 6

Herstellung von Tabletten enthaltend 165 mg Stearyl-ethylfumarat und 80 mg Fumarsäure-monoethylester Calciumsalz

16,5 kg Ethyl-stearyl-fumarat sowie 8,0 kg Fumarsäure-monoethylester-Ca-Salz werden zerkleinert, gemischt und mittels eines Siebes 800 homogenisiert. Es wird ein Hilfsstoffgemisch folgender Zusammensetzung hergestellt: 5.4 kg mikrokristalline Cellulose (Avicel [R]), 11,0 kg Tablettierhilfsstoff T (Merck) bestehend aus 37,5 % Cellulose, 6,25 % Natrium-Carboxymethylcellulose, 18,75 % kolloidaler Kieselsäure (Aerosil[R] 200), 6,25 % Magnesiumstearat und 31,25 % Stärke sowie 5,0 kg Calciumhydrogenphosphatdihydrat ( $CaHPO_4$, Emcompress [R]), 3,3 kg Polyvinylpyrrolidon (Kollidon CL [R]). Das gesamte Pulvergemisch wird mittels eines Siebes 200 homogenisiert und mit einer 2%igen wäßrigen Lösung von Polyvinylpyrrolidon (Kollidon[R] K 30) auf übliche Weise zu einem Bindemittelgranulat verarbeitet und in getrocknetem Zustand mit der äußeren Phase gemischt. Diese besteht aus 0,7 kg eines sog. FST-Komplexes enthaltend

80 % Talk, 10 % kolloidale Kieselsäure und 10 % Magnesiumstearat.

Es wird anschließend auf übliche Weise zu Tabletten von 500 mg Gewicht und 10 mm Durchmesser mit Kreuzkerbe gepreßt.

Anstelle dieser klassischen Tablettiermethode können auch andere Methoden zur Herstellung von Tabletten angewendet werden, wie Direkt tablettierung, sowie feste Dispersionen der Prodrugs nach der Schmelzmethode und der Sprühtrocknungsmethode.

Beispiel 7

Herstellung von Tabletten enthaltend 237 mg Dodecyl-hydrogenfumarat und 80 mg Fumarsäure-monoethylester Calcium Salz

23,7 kg Dodecyl-hydrogenfumarat und 8,0 kg Fumarsäure-monoethylester Calcium Salz werden zerkleinert, gemischt und mittels eines Siebes 800 homogenisiert. Hierauf wird in einem innigen Gemisch von 6,0 kg mikrokristalliner Cellulose (Avicel $^{R}$), 11,5 kg Tablettierhilfs stoff 1 (Merck, siehe Beispiel 10), 5,0 kg Calciumhydrogenphosphatdihydrat (Emcompress $^{R}$), 3,6 kg Polyvinylpyrrolidon (Kollidon$^{R}$ K 30) das Wirkstoffgemisch homogen untermischt, durch ein Sieb 200 geschlagen und mit einer 2%igen wässrigen Lösung von Polyvinylpyrrolidon (Kollidon$^{R}$ K 30) auf übliche Weise zu einem Bindemittelgranulat aufgearbeitet, dem 0,7 kg eines sog. FST-Komplexes als äußere Phase beigemischt werden. Es wird zu Tabletten von 585 mg Gewicht und 12 mm Durchmesser mit Kreuzkerbe komprimiert. Neben den Bindemittelmethoden können ebenfalls andere Tablettiermethoden gemäß Beispiel 10 Verwendung finden.

Beispiel 8

Herstellung einer 0/W Emulsionssalbe, enthaltend 2 % 1,3-Dipalmitoyl-2-Glycerolethfumarat (Vollester)

Zur Herstellung einer gut spreitbaren, kühlenden und abwaschbaren 0/W Emulsionssalbe werden 2,0 kg 1,3-Dipalmitoyl-2-Glycerol-ethfumarat in einer Schmelze eines Gemisches enthaltend 4,3 kg Cetylan$^{R}$ und 25,5 kg hydriertem Arachidoel (Ph.Helv.VI) bei 70° C inkorporiert. Andererseits wird eine Lösung von 2,0 kg Allantoin (5-Ureidohydantoin) in einem Gemisch von 8,6 kg Propylenglykol, 10,0 kg L (+) Milchsäure und 47,6 kg Wasser bei 70° C gelöst.

Die wässrige Lösung wird der öligen Schmelze portionsweise inkorporiert, das Ganze emulgiert und kalt gerührt. Kurz vor dem Erstarren wird bei ca. 30° C homogenisiert und die 0/W-Salbe anschließend in entsprechende Tuben abgefüllt.

Beispiel 9

Herstellung eines Hydrogels mit 3,9 % Octyl-hydrogenfumarat (Halbester)

7,8 kg Monooctyl-fumarat wird mit einem Gemisch von 20,0 kg Kokosfettsäure-Diethanolamid (Comperlan KD $^{R}$), 50,0 kg Natriumlaurylethersulfat Texapon H 25 $^{R}$), 4,0 kg Harnstoff verrieben und in das innige Gemisch portionenweise insgesamt 100,0 kg Wasser von 40° C eingearbeitet.

Anschließend wird mit wäßriger offizineller Pufferlösung von pH 5,5 der Ph.H.VI. bestehend aus Na0H-Essigsäure aufeinen pH von 4,4 eingestellt und mit Wasser auf insgesamt 200,0 kg Masse verdünnt. Das auf Zimmertemperatur gekühlte, leicht gelbliche aber klare, durchsichtige Hydrogel wird in Tuben zu je 10 g abgefüllt. Dieses Hydrogel eignet sich sowohl als antipsoriatisches Douchegel wie als Haarshampoo.

Beispiel 10

Herstellung einer Pinsel lösung mit 6,3 % Dioctylfumarat (Vollester) für keratinöse Körperstellen (Finger-und Zehennägel, sowie Haarbasis)

In 90,0 kg eines Lösungsmittelgemisches von 40 Vol% Essigester und 60 Vol% Methylethylketon werden 6,3 kg Dioctyl-fumarat sowie 3,3 kg Polyacrylharz (Eudragit E 100 R) als Filmbildner und 0,4 kg Dibutylphthalat als Weichmacher gelöst.

Diese Pinsel lösung eignet sich als Psoriatikum für keratinöse Hautstellen, wie Haar, Finger- und Zehennägel. Sie ist hinsichtlich Klebrigkeit, Abrieb, Abwaschbarkeit, Trocknungseigenschaften, Viskosität, Spreitung und Haftfähigkeit optimiert.

Die nachfolgend aufgeführten Verbindungen können gemäß den jeweils angegebenen und vorstehend ausführlich beschriebenen Beispielen erhalten werden.

Verbindungen der allgemeinen Formel (I) gemäß den Beispielen 1 bis 4:

Propyl-hydrogenfumarat, Hexyl-hydrogenfumarat, Octyl-hydrogenfumarat, Dodecyl-hydrogenfumarat, Hexadecyl-hydrogenfumarat, Octadecyl-hydrogenfumarat, Eicosyl-hydrogenfumarat, Ethyldodecylfumarat, Ethylhexadecylfumarat, Methyloctadecylfumarat, Ethyloctylfumarat.

Folgende Angaben sollen dazu dienen, dem Fachmann die Formulierung der erfindungsgemäßen Präparate beispielhaft zu zeigen:

| | |
|---|---|
| Dosierungsangaben: | (bei Psoriasis) |
| peroral: | je nach Indikation ca. 600 bis 850 mg täglich, bezogen auf freie Fumarsäure |
| topisch: | (Salben und Crêmen, Hydrogele) Applikation je nach Indikation 2 bis 4 mal täglich auftragen |

Hilfsstoffe:

Zusammenstellung der üblicherweise zur Verformung verwendeten Hilfsstoffe:

| | |
|---|---|
| Peroral: | (Tabletten bzw. Hartgelatinekapseln) Lactose, mikrokristalline Cellulose (Avicel)R Na-Carboxymethylcellulose, koll. Kieselsäure (Aerosil)R Magnesiumstearat, versch. Stärkesorten, $CaHPO_4$, Polyvinylpyrrolidon (PVP) |
| Parenteral: | die üblichen Elektrolyte, TweenR20 bis TweenR80, SpanR20 bis Span R80, N-Laurylsulfat, PVP (Kollidon) niedrig viskose synthetische Öle wie Isopropylpalmitat, -myristat, Ethyloleat, etc. PluronicRF 68, Wasser |
| Topisch: | neben den unter Parenteralia aufgeführten Hilfsstoffen zusätzlich 5-Ureidohydantoin, hydr. Arachidöl, CetylanR, Propylenglykol, Glycerin, Milchsäure, ComperlanRKD, TexaponR N 25 |

für Haare, Finger- und Zehennägel: Essigester, Methylethylketon, EudragitR E 100 (Filmbilner) Dibutylphthalat (Weichmacher)

Indikationen: Psoriasis (Schuppenflechte) alle Formen; antimikrobielles Therapeuticum

Beispiele für erfindungsgemäße Formulierungen:

Formulierungen

1. Ethylhexadecylfumarat Kapseln

| | |
|---|---|
| Ethylhexadecylfumarat | 150 mg |
| Monoethylfumarat Ca-Salz | 80 mg |

2. Monododecylfumarat Tabletten

| | |
|---|---|
| Dodecylhydrogenfumarat | 294 mg |

3. Monohexadecylfumarat Tabletten

| | |
|---|---|
| Monohexadecylfumarat | 352 mg |

4. Monooctylfumarat-Filmtabletten

| | |
|---|---|
| Monooctylfumarat | 284 mg |
| Monoethylfumarat-ZnSalz | 3 mg |
| Monoethylfumarat-Mg-Salz | 5 mg |

Im folgenden wird die Behandlung dreier Patienten und ihr Ergebnis beschrieben:

LR* - männlich - 1931

- 172 cm
- 82,5 kg
- Krankheit Psoriasis
- anfangs Fumaderm mite** Tabletten
  - Dosis: 3 x 1 Tabl. täglich
  - Dauer: 2 Wochen
  - Flush; Magen-Darm-Beschwerden
- Produg: Ethylhexadecylfumarat Kapsel
  - Dosis: 3 x 1 Kapsel täglich
  - Dauer: 3 Monate
  - kein Flush; Verträglichkeit gut
  - nach 4 Wochen 75 % Psoriasis geheilt
  - Laborkontrolle normal

AF* - weiblich - 1967

- 152 cm
- 61 kg
- Krankheit Psoriasis
- anfangs Fumaderm mite** Tabletten
  - Dosis: 2 x 1 Tabl. täglich
  - Dauer: 3 Monate
  - Flush; Magen-Darm-Beschwerden
- Produg: Monohexadecylfumarat Tabletten
  - Dosis: 3 x 1 Tabl. täglich
  - Dauer: 4 Monate
  - kein Flush; Verträglichkeit gut
  - 50 % Psoriasis geheilt
  - Laborkontrolle normal

NK*- weiblich - 1934

- 160 cm
- 70 kg
- Krankheit Psoriasis
- anfangs Fumaderm mite® Tabletten
  - Dosis: 2 x 1 Tabl. täglich
  - Dauer: 6 Monate
  - Flush; Magern-Darm-Beschwerden

- Produg: Monododecylfumarat Tabletten
  - Dosis; 3 x 2 Tabl. täglich
  - Dauer: 4 Monate
  - kein Flush; Verträglichkeit gut
  - 50 % Psoriasis geheilt
  - Laborkontrolle normal

* Alle drei Patienten waren für länger als 7 Jahre an Psoriasis erkrankt, und vor der Fumarsäuretherapie wurden sie ohne Erfolg mit Corticosteroid topisch behandelt.
Wegen der Magen-Darm-Beschwerden wurde nur Fumaderm mite[R] (schwächere Dosis) versucht
Laborkontrolle: Kreatininspiegel; Blutbild; Transaminase und Urinstatus.

## Ansprüche

1. Fumarsäurederivate der allgemeinen Formel (I)

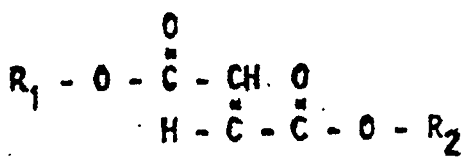

(I)

in der
$R_1$ ungleich $R_2$ ist (unsymmetrisch) und
$R_1$ eine $C_1$ - $C_8$-Alkylgruppe oder ein metallisches Kation wie zu Beispiel Ca, Zn und (außer Ca-Salz von Verbindungen, wo $R_2$ $C_{14}$, $C_{16}$ oder $C_{18}$ ist)
$R_2$ eine gesättigte oder ungesättigte aliphatische $C_6$ - $C_{24}$-Kohlenwasserstoffgruppe bedeuten.

2. Fumarsäurederivate der allgemeinen Formeln (II), (III), (IV) oder (V), basierend auf einem Glycerol-, Alkandiol oder Polyolmolekül der allgemeinen Formeln

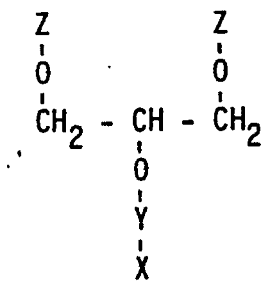

(II)

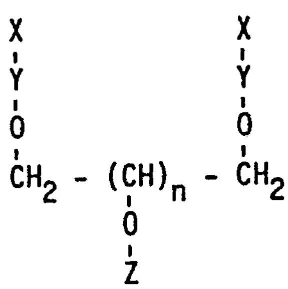

(III)

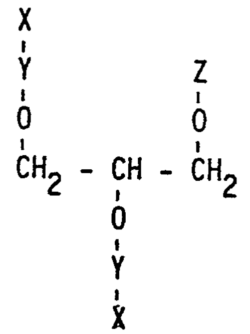

(IV)

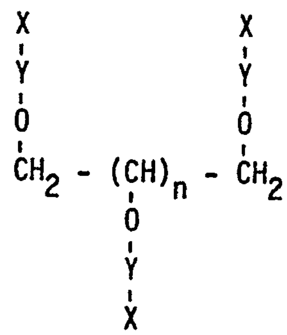

(V)

in denen n die Zahl 1 bis 3, Y die Fumarestergruppe der Formel (VI)

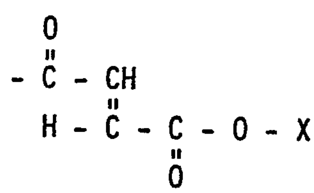

(VI)

X ein Wasserstoffatom, eine $CH_3$-, $C_2H_5$-Gruppe oder ein metallisches Kation,
Z eine gesättigte oder ungesättigte $C_6$ - $C_{24}$-Acylgruppe der Formel (VII)

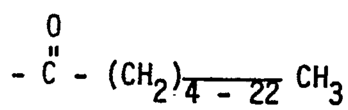

(VII)

eine Gruppierung der Formel (VIII)

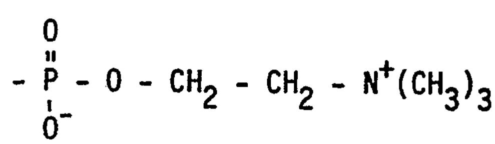

(VIII)

oder ein Wasserstoffatom und 0Z ein Wasserstoffatom oder eine $C_1$ - $C_8$- Alkylgruppe bedeuten.

**3.** Fumarsäurederivate der allgemeinen Formel (IX)

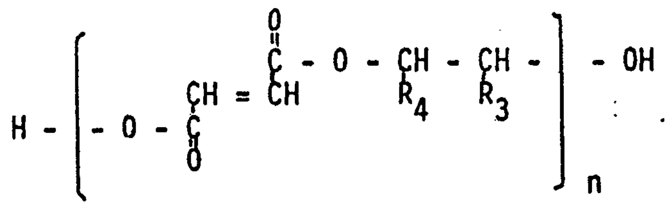 (IX),

in der
n die Zahl 30 bis 260,
$R_3$ ein Wasserstoffatom oder eine $C_1$ - $C_3$-Alkylgruppe,
R4 eine der Gruppen $R_3$ und n die Zahl der Molekylwiederholungen bedeuten.

**4.** Verfahren zur Herstellung von Fumarsäurederivaten gemäß Anspruch 1, dadurch gekennzeichnet, daß man eine Verbindung der allgemeinen Formel (X)

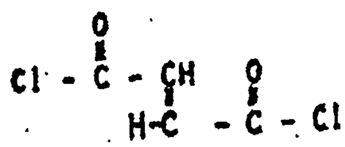 (X),

a) mit 2 Mol Alkylalkohol ( $R_2OH$ ) zum Diester kondensiert und anschließend kontrolliert zum Monoester hydrolisiert, oder
b) mit 1 Mol eines entsprechenden Alkylalkohols ( $R_2OH$ ) kondensiert und das erhaltene Monosäurechlorid zur Säure hydrolisiert oder
c) Fumarsäure direkt mit 2 Mol Alkylalkohol ( $R_2OH$ ) gemäß Anspruch 1 zu einem Diester kondensiert und anschließend kontrolliert zum Monoester hydrolisiert, oder
d) der in a) b) und c), hydrolisierte Monoester wird zu den in Anspruch 1 aufgeführten Salzen umgesetzt.

**5.** Verfahren zur Herstellung von Fumarsäurederivaten gemäß Anspruch 1, dadurch gekennzeichnet, daß man eine Verbindung der allgemeinen Formel (XI)

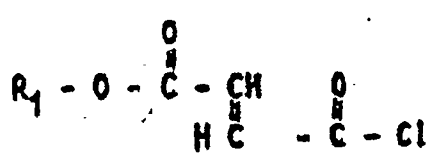 (XI),

in der $R_1$ die in Anspruch 1 angegebene Bedeutung hat, mit einem Mol eines entsprechenden Alkylalkohols ($R_2OH$) unter Bildung eines unsymmetrischen Diesters der Gruppierung $R_2$ gemäß Anspruch 1 kondensiert.

**6.** Verfahren zur Herstellung von Fumarsäure-Verbindungen der allgemeinen Formel (II), (III), (IV) und (V) nach Anspruch 2, **dadurch gekennzeichnet,** daß man eine Verbindung der allgemeinen Formel (XII)

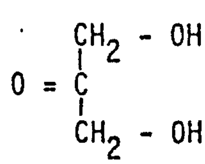 (XII),

mit einem Carbonsäurechlorid der allgemeinen Formeln (XIII) oder (XIV)

Z - Cl (XIII),

X - Y - Cl (XIV),

in denen Z, X und Y die in Anspruch 2 angegebenen Bedeutungen haben, zur Umsetzung bringt, die erhaltenen Carbonyl-Verbindungen zu den entsprechenden Hydroxy-Verbindungen reduziert und gegebenenfalls mit Carbonsäurechlorid der allgemeinen Formel (XIV) oder (XIII) zur Umsetzung bringt.

7. Verfahren zur Herstellung von Fumarsäurederivaten der allgemeinen Formeln (II), (III), (IV) und (V) **dadurch gekennzeichnet,** daß man Glycerin, Alkandiol oder Polyol mit einem Carbonsäurechlorid der allgemeinen Formel (XIII) und (XIV) zur Umsetzung bringt, wobei X, Y und Z die in Anspruch 2 angegebenen Bedeutungen haben.

8. Verfahren zur Herstellung von Fumarsäurederivaten der allgemeinen Formel (IX) nach Anspruch 3, **dadurch gekennzeichnet,** daß man ein Mol Fumarsäure mit einem Mol einer Verbindung der allgemeinen Formel (XV)

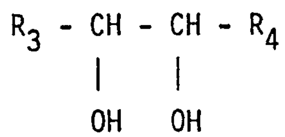

zu einem Polyester kondensiert, wobei $R_3$ und $R_4$ die in Anspruch 3 angegebenen Bedeutungen hat.

9. Pharmazeutische Zubereitungen, enthaltend Fumarsäurederivate gemäß Anspruch 1 für die orale Verabreichung in Form von Kapseln, Granulaten und Tabletten für die parenterale Verabreichung in Form wässriger Mikro-Dispersionen, 0/W Emulsionen oder ölige Lösungen für die rektale Verabreichung als Suppositorien oder Mikroklistiere.

10. Pharmazeutische Zubereitungen, enthaltend Fumarsäurederivate der allgemeinen Formeln (II) bis (V) und (IX) für die perorale Verabreichung in Form von Kapseln, Granulaten und Tabletten für die kutane und transdermale Verabreichung in Form von Salben, Pflastern, Lotionen und Duschmitteln, für die parenterale Verabreichung in Form wässriger Mikro-Disperionen, 0/W-Emulsionen oder ölige Lösungen für die rektale Verabreichung als Suppositorien oder Mikroklistiere, sowie für die medikamentöse Behandlung von Haaren, Finger- und Zehennägeln.

11. Pharmazeutische Zubereitungen, enthaltend Fumarsäurederivate der allgemeinen Formel (I)

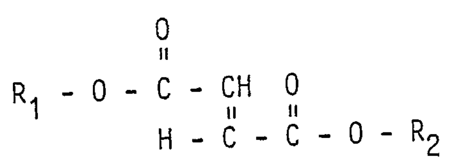

(I)

in der

$R_1$ ein Wasserstoffatom, eine $C_1$ - $C_8$-Alkylgruppe oder ein metallisches Kation wie zum Beispiel Na, Ca, Zn und

$R_2$ eine gesättigte oder ungesättigte aliphatische $C_6$ - $C_{24}$-Kohlenwasserstoffgruppe bedeutet.

12. Pharmazeutische Zubereitung enthaltend Fumarsäurederivate gemäß Anspruch 11 für die kutane und transdermale Verabreichung gegen Psoriasis in Form von Salben, Pflastern, Lotionen und Duschmitteln,

sowie für Haare, Finger- und Zehennägel.

## Claims

1. Fumaric acid derivatives of the general formula (I)

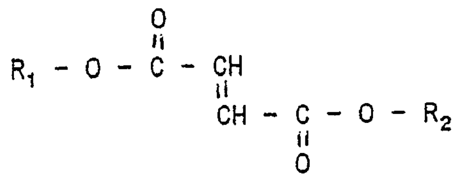

(I)

in which
$R_1$ is not the same as $R_2$ (unsymmetrical), and
$R_1$ means a $C_1$ to $C_8$ alkyl group or a metallic cation such as for instance Ca, Zn (except Ca salts of compounds where $R_2$ is $C_{14}$, $C_{16}$ or $C_{18}$,) and
$R_2$ means a saturated or unsaturated aliphatic $C_6$ to $C_{24}$ hydrocarbon group.

2. Fumaric acid derivatives of the general formulae (II), (III), (IV) or (V), based on a glycerol molecule, alkane diol molecule or a polyol molecule of the general formulae

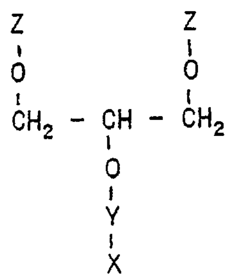

(II)

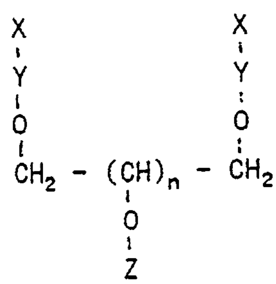

(III)

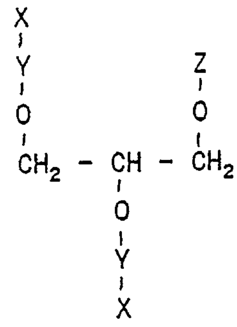

(IV)

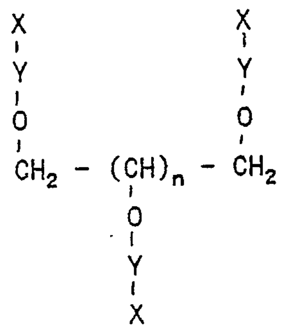

(V)

in which n is a number from 1 to 3,
Y is the fumaric ester group of the formula (VI)

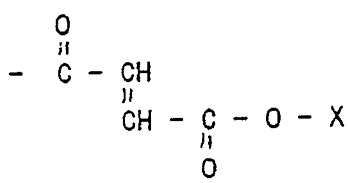

(VI)

X is a hydrogen atom, a $CH_3$ - group, a $C_2H_5$ - group or a metallic cation,
Z is a saturated or unsaturated $C_8$ to $C_{24}$ acyl group of the formula (VII)

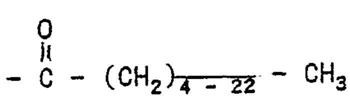

(VII)

or a grouping of the formula (VIII)

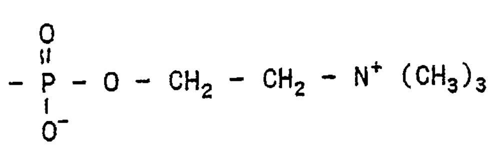

(VIII)

or a hydrogen atom, and
OZ is a hydrogen atom or a $C_1$ to $C_8$ alkyl group.

**3.** Fumaric acid derivatives of the general formula (IX)

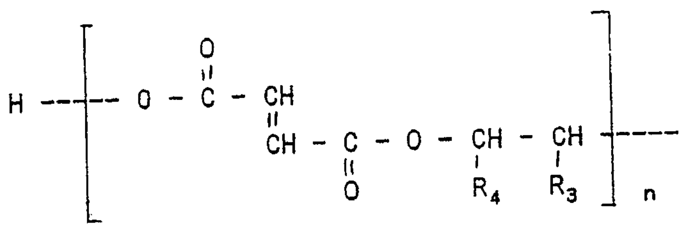

(IX)

in which

n is a number from 30 to 260,
$R_3$ is a hydrogen atom or a $C_1$ to $C_3$ alkyl group,
$R_4$ is one of the groups $R_3$, and
n is the number of molecular repeats.

**4.** Process for the preparation of fumaric acid derivatives according to Claim 1, characterised in that, a compound of the general formula (X)

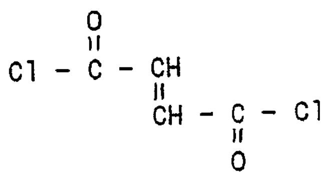

(X)

(a) is condensed with 2 mol alkyl alcohol ( $R_2OH$ ) to give the diester and is then hydrolysed in a controlled way to give the mono-ester, or
(b) is condensed with 1 mol of the respective alkyl alcohol ( $R_2OH$ ) and the monoacid chloride so obtained is hydrolysed to the acid, or
(c) fumaric acid is condensed directly with 2 mol of alkyl alcohol ( $R_2OH$ ) according to Claim 1 to give a diester and is then hydrolysed in a controlled way to give mono-ester, or
(d) the mono-ester hydrolysed in (a) (b) or (c) is reacted to give the salts cited in Claim 1.

**5.** Process for the preparation of fumaric acid derivatives according to Claim 1, characterised in that, a compound of the general formula (XI)

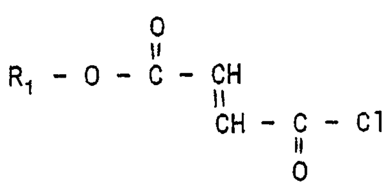

(XI)

in which $R_1$ has the meaning given in Claim 1, is condensed with one mol of a respective alkyl alcohol ( $R_2OH$ ) to give an unsymmetrical diester of the group $R_2$ according to Claim 1.

**6.** Process for the preparation of fumaric acid compounds of the general formulae (II), (III), (IV) and (V) in accordance with Claim 2, characterised in that, a compound of the general formula (XII)

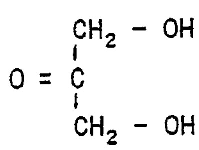

(XII)

is reacted with a carboxylic acid chloride of the general formulae (XIII) or (XIV)

$$Z - Cl \qquad (XIII)$$

$$X - Y - Cl \qquad (XIV)$$

in which Z, X and Y have the meanings cited in Claim 2, and the carbonyl compound so obtained is reduced to the corresponding hydroxy compound and optionally is reacted with carboxylic acid chloride of the general formulae (XIV) or (XIII).

7. Process for the preparation of fumaric acid derivatives of the general formulae (II), (III), (IV) and (V), characterised in that, glycerol, alkane diol or polyol is reacted with a carboxylic acid chloride of the general formulae (XIII) and (XIV) where X, Y and Z have the meanings cited in Claim 2.

8. Process for the preparation of fumaric acid derivatives of the general formula (IX) in accordance with Claim 3, characterised in that, one mol of fumaric acid is condensed with one mol of a compound of the general formula (XV)

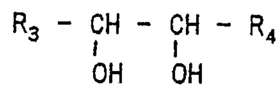

to give a polyester where $R_3$ and $R_4$ have the meanings cited in Claim 3.

9. Pharmaceutical preparations containing fumaric acid derivatives according to Claim 1 for oral administration in the form of capsules, granules and tablets, for subcutaneous administration in the form of aqueous micro-dispersions, 0/W emulsions or solutions in oils and for rectal administration as suppositories or with micro-applicators.

10. Pharmaceutical preparations containing fumaric acid derivatives of the general formulae (II) to (V) and (IX) for oral administration in the form of capsules, granules and tablets, for cutaneous administration in the form of ointments, plasters, lotions and douches, for subcutaneous administration in the form of aqueous micro-dispersions, 0/W emulsions or solutions in oils, for rectal administration as suppositories or with micro-applicators, and also for the medicinal treatment of hair, finger-nails and toe-nails.

11. Pharmaceutical preparations Containing fumaric acid derivatives of the general formula (I)

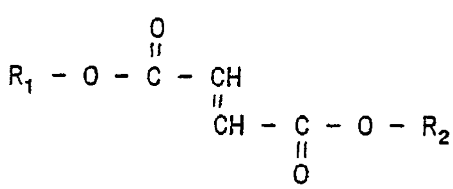

(I)

in which

$R_1$ is a hydrogen atoms, a $C_1$ to $C_8$ alkyl group or a metallic cation such as for example Na, Ca, Zn, and

$R_2$ is a saturated or unsaturated aliphatic $C_8$ to $C_{24}$ hydrocarbon group.

12. Pharmaceutical preparation containing fumaricacid derivatives according to Claim 11 as a treatment for psoriasis by cutaneous administration in the form of ointments plasters, lotions and douches, and also for hair, finger-nails and toe-nails.

## Revendications

**1.** Dérivés de l'acide fumarique de formule générale (I)

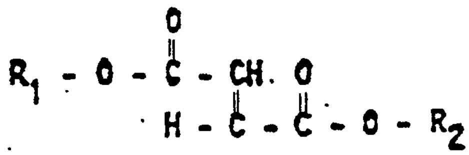

(I)

dans laquelle

$R_1$ est différent de $R_2$ (asymétrique) et

$R_1$ est un groupement alkyle de $C_1$ à $C_8$ ou un cation métallique tel que Ca, Zn et (à l'exception du sel de calcium de composés dans lesquels $R_2$ est un groupement en $C_{14}$, $C_{16}$ ou $C_{18}$)

$R_2$ est un groupement hydrocarburé aliphatique saturé ou insaturé comportant 6 à 24 atomes de carbone.

**2.** Dérivés de l'acide fumarique des formules générales (II), (III), (IV) ou (V), sur base d'une molécule de glycérol, alcanediol ou polyol des formules générales

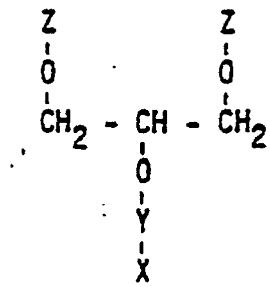

(II)

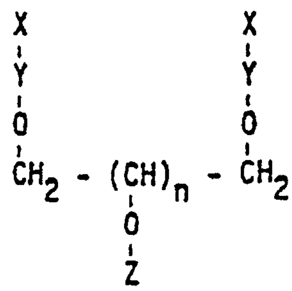

(III)

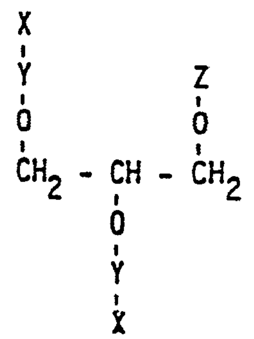

(IV)

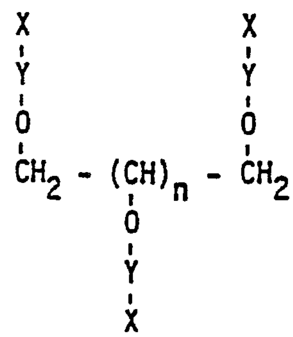

(V)

dans lesquelles n est un nombre de 1 à 3, Y correspond au groupement d'ester fumarique de formule (VI)

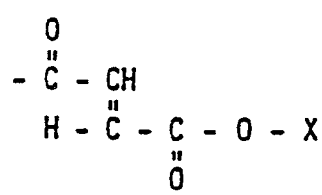

(VI)

X représente un atome d'hydrogène, un groupement $CH_3$-, $C_2H_5$ ou un cation métallique,
Z est un groupement acyle saturé ou insaturé de $C_6$ à $C_{24}$ de formule (VII)

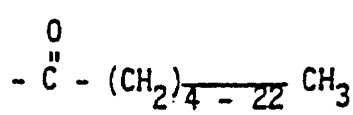

(VII)

un groupement de formule (VIII)

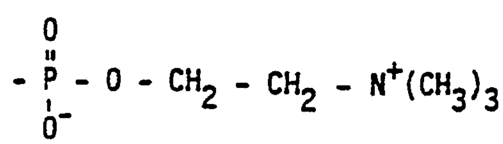

(VIII)

ou un atome d'hydrogène, et OZ représente un atome d'hydrogène ou un groupement alkyle de $C_1$ à $C_8$.

**3.** Dérivés de l'acide fumarique de formule générale (IX)

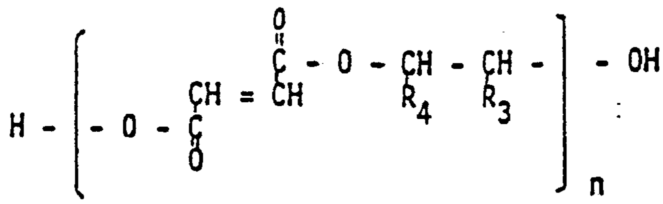

(IX),

dans laquelle

n est un nombre compris entre 30 et 260,

$R_3$ représente un atome d'hydrogène ou un groupement alkyle de $C_1$ à $C_3$,

$R_4$ correspond à un des groupements $R_3$ et n représente le nombre de répétitions de la molécule.

4. Procédé de fabrication de dérivés de l'acide fumarique conformes à la revendication 1, caractérisé en ce qu'un composé de formule générale (X)

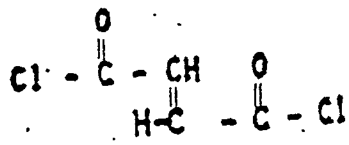

(X),

a) est condensé en diester à l'aide de 2 moles d'alkylalcool ( $R_2OH$ ) et ensuite hydrolysé de manière contrôlée en monoester, ou bien

b) est condensé à l'aide de 1 mole d'un alkylalcool correspondant ( $R_2OH$ ) et le chlorure de monoacide obtenu est hydrolysé en acide, ou bien

c) l'acide fumarique est condensé directement en diester à l'aide de 2 moles d'alkylalcool ( $R_2OH$ ), conformément à la revendication 1, pour elle ensuite hydrolysé de manière contrôlée en monoester, ou bien

d) le monoester hydrolysé en a), b) et c) est transformé en sels énumérés dans la revendication 1

5. Procédé de fabrication de dérivés de l'acide fumarique conformes à la formule (I) ci-dessus, dans lequel un composé de formule générale (XI),

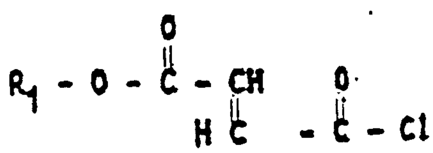

(XI),

dans laquelle $R_1$, possède la valeur indiquée dans la revendication 1, est condensé à l'aide d'une mole d'un alkylalcool correspondant ( $R_2OH$ ) pour former un diester asymétrique du groupement $R_2$ conforme à la revendication 1.

6. Procédé de fabrication de dérivés de l'acide fumarique des formules générales (II), (III), (IV) et (V) selon la revendication 2, caractérisé en ce que l'on fait réagir un composé de formule générale (XII)

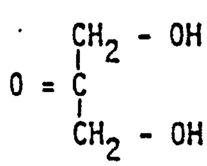

(XII),

avec un chlorure d'acide carboxylique des formules générales (XIII) ou (XIV)

$$Z - Cl \qquad (XIII),$$

$$X - Y - Cl \qquad (XIV),$$

dans lesquelles Z, X et Y possèdent la signification indiquée dans la revendication 2, que l'on réduit les composés carbonyle obtenus en composés hydroxy correspondants et en ce que on fait réagir éventuellement ces derniers avec du chlorure d'acide carboxylique des formules générales (XIV) ou (XIII).

7. Procédé de fabrication de dérivés de l'acide fumarique des formules générales (II), (III), (IV) et (V), caractérisé en ce que l'on fait réagir de la glycérine, un alcanediol ou un polyol avec un chlorure d'acide carboxylique des formules générales (XIII) et (XIV), dans lesquelles X, Y et Z possèdent la signification mentionnée dans la revendication 2.

8. Procédé de fabrication de dérivés de l'acide fumarique de formule générale (IX) conformes à la revendication 3, caractérisé en ce que l'on condense en un polyester une mole d'acide fumarique avec une mole d'un composé de formule générale (XV)

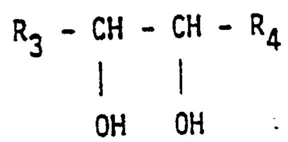

(XV)

dans lequel $R_3$ et $R_4$ possèdent la signification mentionnée dans la revendication 3.

9. Préparations pharmaceutiques renfermant des dérivés de l'acide fumarique conformes à la revendication 1, destinées à l'administration orale, sous la forme de gélules, granulés et comprimés, à l'administration parentérale sous la forme de micro-dispersions aqueuses, d'émulsions huile dans l'eau ou de solutions huileuses, à l'administration rectale sous la forme de suppositoires ou de micro-lavements.

10. Préparations pharmaceutiques renfermant des dérivés de l'acide fumarique des formules générales (II) à (V) et (IX), destinées à l'administration orale, sous la forme de gélules, granulés et comprimés, à l'application cutanée et à l'administration transdermique sous la forme de pommades, emplâtres, lotions et produits pour la douche, à l'administration parentérale sous la forme de micro-dispersions aqueuses, d'émulsions huile dans l'eau ou de solutions huileuses, à l'administration rectale sous la forme de suppositoires ou de micro-lavements, ainsi que qu'au traitement médicamenteux des cheveux, et des ongles des doigts et des pieds.

11. Préparations pharmaceutiques renfermant des dérivés de l'acide fumarique de formule générale (I)

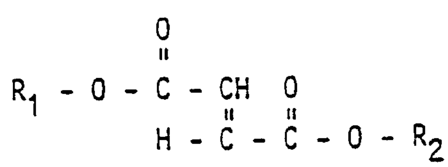

(I)

dans laquelle

$R_1$ représente un atome d'hydrogène, un groupement alkyle de $C_1$ à $C_8$ ou un cation métallique, tel que Na, Ca, Zn

$R_2$ correspond à un groupement hydrocarburé aliphatique saturé ou insaturé de $C_6$ à $C_{24}$.

12. Préparations pharmaceutiques renfermant des dérivés de l'acide fumarique conformes a la revendication 11, destinées à l'application cutanée et à l'administration transdermique pour traiter le psoriasis, sous la forme de pommades, emplâtres, lotions et produits pour la douche, ainsi que pour le traitement des cheveux et des ongles des doigts et des orteils.